# EUROPÄISCHE PATENTANMELDUNG

(11) **EP 4 656 212 A1**
(43) Veröffentlichungstag der Anmeldung: **03.12.2025**
(21) Anmeldenummer: 24178226.7
(22) Anmeldetag: 27.05.2024
(51) Int. Cl.: A61L 27/22, A61L 27/24, A61L 27/50

(54) **EINRICHTUNG UND HERSTELLUNGSPROZESS VON BIOARTIFIZIELLEN KONSTRUKTEN**

(71) Anmelder: Medizinische Hochschule Hannover, 30625 Hannover (DE)
(72) Erfinder: Aper, Thomas, 30629 Hannover (DE); Wilhelmi, Mathias, 30916 Isernhagen (DE)
(74) Vertreter: Kröncke, Rolf

(57) **Zusammenfassung**

Die vorliegende Erfindung richtet sich in einem ersten Aspekt auf eine Einrichtung zur Herstellung von bioartifiziellen Konstrukten auf Proteinbasis, wie auf Kollagenbasis und/oder Fibrinbasis durch ein Rotationsverfahren unter Verwendung einer äußeren Hülle und inneren Form, wobei die äußere Hülle Bohrungen aufweist und die innere Form mindestens zweiteilig ausgebildet ist. In einem weiteren Aspekt richtet sich die vorliegende Erfindung auf ein Verfahren zur Herstellung von bioartifiziellen Konstrukten auf Proteinbasis, insbesondere Kollagen- und/oder Fibrinbasis unter Verwendung der erfindungsgemäßen Einrichtung und auf solche bioartifiziellen Konstrukte erhältlich mit dem erfindungsgemäßen Verfahren. Weiterhin wird eine Vorrichtung zur Herstellung der bioartifiziellen Konstrukte mit Hilfe der erfindungsgemäßen Einrichtung bereitgestellt. Schließlich richtet sich die vorliegende Anmeldung auf eine Fibrinogenlösung, die insbesondere für die erfindungsgemäße Herstellung der bioartifiziellen Konstrukte geeignet ist.

## Beschreibung

Die vorliegende Erfindung richtet sich in einem ersten Aspekt auf eine Einrichtung zur Herstellung von bioartifiziellen Konstrukten auf Proteinbasis, wie auf Kollagenbasis und/oder Fibrinbasis durch ein Rotationsverfahren unter Verwendung einer äußeren Hülle und inneren Form, wobei die äußere Hülle Bohrungen aufweist und die innere Form mindestens zweiteilig ausgebildet ist. In einem weiteren Aspekt richtet sich die vorliegende Erfindung auf ein Verfahren zur Herstellung von bioartifiziellen Konstrukten auf Proteinbasis, insbesondere Kollagen- und/oder Fibrinbasis unter Verwendung der erfindungsgemäßen Einrichtung und auf solche bioartifiziellen Konstrukte erhältlich mit dem erfindungsgemäßen Verfahren. Weiterhin wird eine Vorrichtung zur Herstellung der bioartifiziellen Konstrukte mit Hilfe der erfindungsgemäßen Einrichtung bereitgestellt. Schließlich richtet sich die vorliegende Anmeldung auf eine Fibrinogenlösung, die insbesondere für die erfindungsgemäße Herstellung der bioartifiziellen Konstrukte geeignet ist.

### Stand der Technik

Tissue Engineering spielt in vielen Bereichen der modernen Medizin eine wichtige Rolle. Unter anderem wird Tissue Engineering genutzt, um Gefäßprothesen bereitzustellen. Solche geeigneten Konstrukte, die auch als bioartifizielle Konstrukte bezeichnet werden, nehmen einen immer größeren Aspekt in der medizinischen Transplantationstechnik ein. Seit längerer Zeit wird bereits auf dem Gebiet des Tissue Engineerings gearbeitet, um körpereigene Materialien, wie Organe, Gefäße oder auch Gewebepatches möglichst gleichwertig durch im Labor hergestellte artifizielle Konstrukte aus biologischen und ggf. körpereigenen Materialien zu ersetzen. Insbesondere spielt dieses auch bei Gefäßprothesen eine Rolle.

Gefäßprothesen sind Implantate, die für therapeutische Zwecke üblicherweise dauerhaft im Körper des Individuums eingebracht werden. Sie dienen z. B. als Ersatz natürlicher Blutgefäße um geschädigte Gefäßabschnitte von Verletzungen, Verengungen oder Blockaden, aber auch Erweiterungen oder bei Transplantationen zu ersetzen. Sie dienen sowohl dem Ersatz von Arterien als auch von Venen.

Bisher wurden Gefäßprothesen meist aus Kunststoffen wie Polyethylenterephthalat (PET) oder Polytetrafluorethylen (PTFE) hergestellt. Gerade bei Ersatz von Arterien und hier von großen Gefäßen, wie der Aorta, sind hohe mechanische Anforderungen an die Gefäßprothese gestellt. Entsprechend wurden bisher meist PET-Prothesen für solche größeren Gefäße, wie Aorta oder innere oder äußere Beckenarterien, eingesetzt, PTFE-Prothesen wurden als Ersatz für kleine Gefäße oder bei Bypass-Anlagen genutzt. Nachteil der synthetischen Kunststoffe in solchen Prothesen ist, dass sie eine unzureichende Biokompatibilität aufweisen. Es besteht das Risiko, dass sich Blutgerinnsel bilden. Auch können sich ihre Oberflächen mit bakteriellem Biofilm infizieren, der nur sehr schwer zu therapieren ist. Entsprechend besteht der Bedarf nach bioartifiziellen Konstrukten, z. B. auf Basis von körpereigenen Molekülen, wie Kollagen, Fibrin etc. Allerdings zeigten z.B. die Fibrinprothesen bisher meist Schwierigkeiten bei hohen Druckbelastungen, z.B. dem Aorten-Blutdruck. In Deutschland werden derzeit ca. 13000 Aorten-Ersatzoperationen durchgeführt, die entsprechende Gefäßprothesen benötigen.

Es wurden bereits verschiedene Verfahren und bioartifizielle Konstrukte beschrieben, die als bioverträgliche Alternativen für synthetische Kunststoffkonstrukte bzw. Prothesen dienen. Allerdings zeigt sich hier, dass diese den hohen mechanischen Belastungen nicht standhalten können

In der WO 2013/037349 und der WO 2013/091867 sind Verfahren zur Herstellung von bioartifiziellen und biologischen Gewebekonstrukten bekannt. Ebenfalls werden Vorrichtungen zur Herstellung dieser und die Verwendung spezifisch gewonnener Zellen für Gewebekonstrukte beschrieben. Tatsächlich sind solche Gewebekonstrukte die Zellen enthalten aus verschiedenen Gründen für einen klinischen Einsatz nicht ideal, sodass Konzepte zu ihrer Herstellung nicht mehr prioritär verfolgt werden. Beispiele hierfür sind hohe Zulassungshürden für den klinischen Einsatz und die nur sehr begrenzte Lagerbarkeit dieser "lebenden" Zell-Matrix Konstrukte. Der primäre Fokus zur Generierung bioartifizieller vaskulärer Prothesen liegt daher auf azellulären bioartifiziellen Gewebekonstrukten.

Die WO 2017/005857 beschreibt Verfahren zur Herstellung von bioartifiziellen, primär azellulären Konstrukten, z. B. auf Fibrinbasis sowie diese Konstrukte selbst. Diese Verfahren zeichnen sich durch Druckbeaufschlagung, z. B. durch das Rotationsverfahren aus, wobei während der Herstellung Flüssigkeiten z. B. über semipermeable Membranen aus der Form abgeführt werden müssen. Ein Problem dieser Konstrukte ist, dass gerade für längere Gefäßprothesen mit Längen von mehr als 10 cm die Homogenität der Gefäße und eine hohe Stabilität dieser nicht gewährleistet ist.

Die WO 2022/090563 A1 schlägt Konstrukte auf Fibrinbasis als Implantate zur Nervenregenerierung vor, in denen Seidenfasern axial in den Konstrukten vorliegen. Die Verwendung solcher Konstrukte z. B. bei Aorten-Prothesen wird ebenfalls vorgeschlagen.

Es zeigte sich allerdings, dass gerade die beschriebenen Herstellungsverfahren und die entsprechenden Einrichtungen hierfür die notwendige Herstellung von Prothesen, wie Fibrinprothesen, mit geeigneten Durchmessern, Gefäßwanddichten und -längen mit hoher Stabilität und Homogenität nicht erlauben.

Aufgabe der vorliegenden Erfindung und der Bereitstellung neuer Einrichtungen und Vorrichtungen, die Gefäßprothesen auf Proteinbasis, wie Kollagenbasis und/oder Fibrinbasis, mit den gewünschten Eigenschaften erlauben, sowie solche Gefäßprothesen selbst und Mittel zur Herstellung dieser.

### Beschreibung der Erfindung

Zur Lösung dieser Aufgaben werden Einrichtungen geeignet zur Herstellung von bioartifiziellen Konstrukten auf Proteinbasis, wie auf Kollagenbasis und/oder Fibrinbasis, durch ein Rotationsverfahren bereitgestellt gemäß Anspruch 1 und entsprechende Verfahren zur Herstellung dieser bioartifiziellen Konstrukte auf Proteinbasis, wie Kollegenbasis und/oder Fibrinbasis, gemäß Anspruch 7 sowie damit erhältliche bioartifizielle Konstrukte gemäß Anspruch 12. Geeignete Vorrichtungen zur Herstellung der bioartifiziellen Konstrukte mit den erfindungsgemäßen Einrichtungen werden gemäß Anspruch 13 bereitgestellt und schließlich Fibrinogen-Lösungen geeignet zur Herstellung der Konstrukte gemäß Anspruch 14.

Eine erfindungsgemäße Einrichtung ist dabei eine Einrichtung geeignet zur Herstellung von bioartifiziellen Konstrukten auf Proteinbasis, wie auf Kollagenbasis oder Fibrinbasis durch ein Rotationsverfahren, mit i) einer äußeren Hülle und ii) einer inneren Form, wobei die innere Form in die äußere Hülle eingeführt werden kann und die innere Form stirnseitig angesetzte, lösbare Abschlusselemente aufweist, die äußere Hülle Bohrungen zum Abführen von Fluiden umfasst und die innere Form mindestens zweiteilig ist und mit Befestigungselementen zum lösbaren Verbinden der mindestens zweiteiligen inneren Form vorliegt, dadurch gekennzeichnet, dass die mindestens zweiteilige innere Form durch die Befestigungselemente derart verbunden vorliegt, dass Fluide durch die in den mindestens zwei schalenförmigen Teilen der inneren Form ausgebildeten Spalten aus dem Inneren der inneren Form in den Zwischenraum zwischen innerer Form und äußerer Hülle fließen können und diese Fluide dann durch die Bohrungen der äußeren Hülle nach außen abgeführt werden können.

Erfindungsgemäß wird dabei unter einem bioartifiziellen Konstrukt auf Proteinbasis ein Konstrukt verstanden, das auf natürlichen Bestandteilen aufgebaut ist. Dieses Konstrukt wird auf Proteinbasis, wie auf Kollagenbasis und/oder Fibrinbasis bereitgestellt. Entsprechend weist dieses bioartifizielle Konstrukt eine Matrix auf dieser Basis auf. Zumindest weist das erfindungsgemäße Konstrukt eine Schicht auf, die im Wesentlichen eine Matrix ist auf Proteinbasis wie auf Kollagenbasis und/oder Fibrinbasis. Unter dem Ausdruck "Matrix" wird dabei eine Struktur verstanden, die durch das entsprechende Protein ausgebildet wird. Üblicherweise ist die Matrix dabei eine provisorische Matrix, d. h. sie löst sich einige Zeit nach Implantation auf und wird durch ein sich parallel dazu vom Körper selbst gebildetes - "Neogefäß" ersetzt.

Die Matrix kann insbesondere eine auf Kollagenbasis, Fibrinbasis oder Mischungen hiervon sein. Eine Ausführungsform betrifft ein bioartifizielles Produkt auf Fibrinbasis. Das in der vorliegenden Anmeldung beschriebene bioartifizielle Konstrukt ist dabei in einer Ausführungsform ein primär azelluläres Konstrukt, d. h. ein Konstrukt, in dem primär keine Zellen vorliegen. Dabei werden die Ausdrücke zellfrei und azellulär synonym verwendet. Als Matrixmaterial wird ein Material bevorzugt, wie es auch in der Natur in einer azellulären Matrix von Geweben vorkommt und auch als inter-/ extrazelluläre Substanz bezeichnet wird. Diese sollte insbesondere eine sein, die vom Körper als immunologisch tolerierbar identifiziert oder sogar aus körpereigenen (autologen) Substanzen stammen.

Entsprechend sind die Konstrukte auf Proteinbasis solche mit auf Basis von natürlichen Proteinen, insbesondere Kollagen oder Fibrin.

Geeignete Matrices sind aus dem Stand der Technik bekannt, z. B. wie in der WO 2017/005857 beschrieben.

Die erfindungsgemäße Einrichtung weist dabei die äußere Hülle auf, die mit Bohrungen ausgestattet ist. Diese durch die Hülle erstreckende Bohrungen erlauben es, Fluide, insbesondere Flüssigkeit aus dem Inneren der äußeren Hülle nach außen außerhalb der äußeren Hülle zu transportieren.

Die äußere Hülle ist aus geeigneten Materialien, wie sie bei Rotationsverfahren eingesetzt werden können, ausgebildet. Der innere Querschnitt der äußeren Hülle ist dabei größer als der äußere Querschnitt der inneren Form, sodass die innere Form in die äußere Hülle eingeschoben werden kann. Mit anderen Worten, der Querschnitt der inneren Form, gebildet aus den mehrteiligen schalenförmigen Teilen ausgebildet, ist im Wesentlichen kreisförmig und der Querschnitt der inneren Form ist kleiner als der Querschnitt der äußeren Form, um das Einschieben der inneren Form in die äußere Form zu ermöglichen. Ein geeignetes Material der äußeren Hülle ist Stahl, andere Materialien sind solche, die die notwendige Stabilität im Rotationsverfahren aufzeigen und insbesondere solche, die eine Sterilisation erlauben, um entsprechende bioartifizielle Konstrukte als Medizinprodukte bereitzustellen.

Die in der äußeren Hülle vorliegenden Bohrungen sind dabei solche, die einen Durchmesser von mindestens 1 mm, bevorzugt mindestens 2 mm, wie mindestens 3 mm oder wie mindestens 5 mm aufzeigen. Insbesondere sind die Bohrungen so ausreichend dimensioniert, dass auch kleine Stücke von Fibrin heraustransportiert werden können und so ein Verstopfen dieser Bohrungen ausgeschlossen wird.

Die innere Form der erfindungsgemäßen Einrichtung ist eine, die mindestens zweiteilig ausgebildet ist, d. h., eine innere Form aus mehreren Teilen. In einer Ausführungsform sind dieses mindestens zwei Teile, d. h. zwei Halbschalen. In einer Ausführungsform sind hierbei diese Teile, z.B. die Halbschalen in Längsrichtung angeordnet, sodass entsprechend mindestens zwei Spalten in Längsrichtung ausgebildet sind, wobei diese Spalten vom Inneren der inneren Form zum Äußeren der inneren Form reichen. Mit anderen Worten, sie stellen einen Durchgangsspalt dar. Diese Durchgangsspalte können durch die entsprechenden schalenförmigen Teile der mindestens zwei schalenförmigen Teile der inneren Form ausgebildet sein und/oder diese Durchgangsspalte(n) können in den schalenförmigen Teilen und Form selbst vorliegen.

Diese mindestens zwei schalenförmigen Teile der mindestens zweiteiligen inneren Form werden dabei mit Befestigungselementen zum lösbaren Verbinden miteinander verbunden. Geeignete Befestigungselemente sind z. B. Schrauben etc.

Die innere Form wird mit stirnseitig angesetzten lösbaren Abschlusselementen abgeschlossen. Diese Abschlusselemente schließen üblicherweise dichtend die innere Form ab. In einer Ausführungsform können diese Abschlusselemente derart ausgebildet sein, dass sie weiterhin dichtend in die innere Form beim Einschieben dieser in die äußere Hülle abschließt. D. h., in einer Ausführungsform weisen die lösbaren Abschlusselemente Dichtungselemente zum Abdichten der inneren Form und Dichtungselemente zum Abdichten der äußeren Hülle auf.

In einer Ausführungsform ist dabei ein Abschlusselement derart ausgebildet, dass dieses eine Durchgangsöffnung zum Einbringen von Fluiden, wie Flüssigkeiten, in das Innere der inneren Form erlaubt.

Die erfindungsgemäße Einrichtung zeichnet sich dadurch aus, dass die bei der inneren Form vorliegenden Durchgangsspalten ein Abführen von Fluiden aus dem Inneren der inneren Form in den Zwischenraum zwischen innerer Form und äußerer Hülle erlaubt. Diese Fluide, wie Flüssigkeiten, können dann durch die in der äußeren Hülle vorhandenen Bohrungen nach außen abgeführt werden.

Die Durchgangsspalten sind dabei in einer Ausführungsform als kapillare Spalten bzw. Kapillarspalten, auch einfach als Kapillaren bezeichnet, vorliegend. Unter dem Ausdruck "Kapillarspalte" wird vorliegend verstanden, dass eine Drainage der in der inneren Form vorliegenden Fluide, insbesondere der eingebrachten Flüssigkeiten, über diesen Kapillarspalt erfolgt. Aufgrund der Dimensionierung dieser Durchgangsspalte werden im Inneren der inneren Form vorliegende Proteine und andere Moleküle insbesondere das Fibrinogen und Fibrin in der inneren Form zurückgehalten, während die Flüssigkeit aus der inneren Form fließen kann. Hierdurch wird ein Verstopfen sowohl dieses Durchgangsspaltes als auch der in der äußeren Hülle vorliegenden Bohrungen vermieden und erlaubt insbesondere eine homogenere Ausbildung und Verteilung des Fibrins im bioartifiziellen Konstrukt.

Die im bisherigen Stand der Technik beschriebenen Verdichtungen, wie z. B. in der WO 2017/005857 oder WO 2022/090562 A1 erlaubten dieses aufgrund des Vorliegens sehr kleiner Bohrungen in der Hülle, die insbesondere durch semipermeable Membran abgeschlossen werden, nicht. Vielmehr zeigte sich hier, dass bei den kleinen Bohrungen in der äußeren Hülle Flüssigkeiten, wie auch Fibrin oder Fibrinogen, austreten, so dass entweder viel Fibrin verloren wurde oder keine ausreichende Verdichtung vorlag. Darüber hinaus verstopften diese kleinen Bohrungen in der äußeren Hülle aufgrund des Fibrins und der laufenden Umsetzung des Fibrinogens zu Fibrin, eine ausreichende Verdichtung erfolgte daher nicht. Mithin zeichneten sich diese Konstrukte durch hohe Abweichungen in der Stabilität und der Homogenität aus.

In einer Ausführungsform sind dabei die innere Form und die äußere Hülle in einer Längsrichtung erstreckend ausgebildet und/oder die innere Form und die äußere Hülle sind röhrenförmig, insbesondere zylindrisch ausgebildet und koaxial angeordnet.

Die Einrichtung ist so ausgebildet, dass sie einem Rotationsverfahren bei der Herstellung der bioartifiziellen Konstrukte unterworfen werden kann.

Insbesondere bei Vorliegen der Kapillarspalten in der inneren Form können Fibrinogen und noch flüssiges Fibrin aufgrund der höheren Viskosität durch diese Kapillarspalten nicht austreten oder nur in sehr geringem Maß austreten, vielmehr verbleiben sie im Inneren der inneren Form. Die Gefahr eines Verstopfens der Öffnungen, wie der Bohrungen, zum Abführen der Fluide, insbesondere der Flüssigkeiten, ist verringert. Die erhaltenen Konstrukte, wie Gefäßprothese, zeichnen sich durch eine geringe Abweichung der Stabilität der Wand und der mechanischen Eigenschaften aus. Die Fibrinkonstrukte sind homogen ausgebildet mit über das gesamte Konstrukt gleichbleibender Wanddicke.

Insbesondere erlaubt die erfindungsgemäße Einrichtung eine standardisierte kontrollierte Drainage überschüssiger Flüssigkeit aus der Form z. B. dadurch, dass auf der einen Seite über größere Bohrungen die Drainage ausreichend gewährleistet ist und auf der anderen Seite durch die Schaffung der kapillären Spalte zwischen dem schalenförmigen Gehäuseteilen der inneren Form und der Verlust von Fibrinogen/Fibrin verringert wird. Eine Automatisierung und Standardisierung des Herstellungsverfahrens, insbesondere eine nutzerunabhängige Möglichkeit zur Herstellung, konnte erreicht werden.

Die schalenförmigen Gehäuseteile der inneren Form und ggf. die Abschlusselemente sind dabei aus geeigneten Materialien, wie geeigneten Kunststoffen, ausgebildet. Ein insbesondere geeigneter Kunststoff ist PEEK (Polyetheretherketon). Andere geeignete Materialien umfassen Teflon, autoklavierbare Kunststoffe im Allgemeinen oder Keramiken.

In einer Ausführungsform sind die Teile der Einrichtung sterilisierbar, um insbesondere zur Herstellung von bioartifiziellen Medizinprodukten geeignet zu sein.

In einem weiteren Aspekt richtet sich die vorliegende Erfindung auf ein Verfahren zur Herstellung von bioartifiziellen Konstrukten auf Proteinbasis, insbesondere Kollagen und/oder Fibrinbasis. Dieses Verfahren beinhaltet die Verwendung der erfindungsgemäßen Einrichtung. Das erfindungsgemäße Verfahren schließt dabei die folgenden Schritte ein:
a) Bereitstellen von proteinhaltigen Zusammensetzungen und Vernetzer-haltigen Zusammensetzungen;
b) Einbringen der proteinhaltigen Zusammensetzungen und der Vernetzer-haltigen Zusammensetzungen in die erfindungsgemäße Einrichtung unter Druckbeaufschlagung dieser Einrichtung, wobei die Druckbeaufschlagung durch Rotation der Einrichtung erfolgt und wobei während des Rotationsverfahrens Fluide über die Bohrungen der äußeren Hülle der erfindungsgemäßen Einrichtung abgeführt werden;
c) Entfernen des so erhaltenen bioartifiziellen Konstruktes auf Proteinbasis, insbesondere Kollagenbasis und/oder Fibrinbasis, aus der inneren Form der erfindungsgemäßen Einrichtung.

In einer Ausführungsform ist dabei das bioartifizielle Konstrukt auf Proteinbasis eines auf Fibrinbasis und die entsprechenden Zusammensetzungen in eine Fibrinogen-haltige Zusammensetzung und eine Thrombin-haltige Zusammensetzung. Insbesondere handelt es sich bei der Fibrinogen-haltigen Zusammensetzung um eine erfindungsgemäße Fibrinogen-haltige Zusammensetzung, wie unten näher ausgeführt. Die Thrombin-haltige Zusammensetzung stellt die oben genannte Vernetzer-haltige Zusammensetzung dar. Bei Nutzung von Kollagen als Protein ist eine Zusammensetzung eine kollagenhaltige Zusammensetzung, während die Vernetzer-haltige Zusammensetzung eine ist, die z B. über die im Kollagen vorhandenen Lysin-Gruppen eine Vernetzung des Kollagens erreicht.

In einer Ausführungsform werden als Matrix Mischungen aus Kollagen und Fibrin verwendet, so dass entsprechende Zusammensetzungen eingesetzt werden. Die Herstellung und Bereitstellung der genannten Zusammensetzungen sind dem Fachmann bekannt. In einer Ausführungsform ist dabei die Fibrinogen-Lösung eine aus einer Mischung umfassend Fibrinogen das frei von weiteren Bestandteilen ist und Fibrinogen enthaltend weitere Bestandteile, wie z. B. Serum Albumin oder andere Proteine, die bei der Ausreinigung des Fibrinogens in der aufgereinigten Fibrinzusammensetzung verbleiben oder auch gezielt beigemengt werden.

Die Druckbeaufschlagung erfolgt durch Rotation, wie sie aus dem Stand der Technik bekannt ist, insbesondere ist die Druckbeaufschlagung eine Rotation, bei der an der Oberfläche des Konstruktes im Inneren der inneren Form mindestens 100 x g, wie mindestens 200 x g, wie mindestens 500 x g vorliegen. Zum Beispiel kann diese bei 800 x g bis 900 x g liegen.

Das erfindungsgemäße Verfahren erlaubt insbesondere die Herstellung von bioartifiziellen Konstrukten mit Wanddicken von mindestens 0,4 mm, wie mindestens 1 mm Wanddicke. Die Konstrukte haben z. B. einen inneren Durchmesser von 6 mm bis 30 mm, wie 7 mm bis 20 mm, wie 8 mm bis 15 mm.

Die Konstrukte können mit Längen von mindestens 10 cm, wie mindestens 20 cm, z. B. mindestens 30 cm, wie mindestens 40 cm, und länger, wie mindestens 50 cm, hergestellt werden.

Das Einbringen der Fibrinogen-haltigen Zusammensetzungen und der Thrombinhaltigen Zusammensetzungen in die erfindungsgemäße Einrichtung kann dabei gemäß bekanntem Verfahren erfolgen. Eine Möglichkeit hierbei ist, dass über die Durchgangsöffnung in mindestens einem Abschlusselement der erfindungsgemäßen Einrichtung ein geeigneter Applikator zum Einbringen der Fluide (Fibrinogen-haltige Zusammensetzung und Thrombin-haltige Zusammensetzung) vorliegt. Dabei können die beiden Zusammensetzungen mit verschieden definierten Flussraten in getrennten Schläuchen zum Applikator geführt werden. Die Durchmischung kann dabei im Applikator selbst oder erst in der Form erfolgen.

In einer Ausführungsform kann dabei das Einbringen der genannten Zusammensetzungen als entsprechende Lösungen über eine Steuerung erfolgen, bei denen die entsprechenden Schritte des Einbringens vorbestimmt ablaufen. Ein entsprechendes Programm umfasst dabei z. B. einzelne Schritte, bei denen definiert sind die Dauer des Schritts, die Rotationsgeschwindigkeit der Form, die Position des Applikators, die jeweilige Applikationsrate der Lösungen von Fibrinogen und Thrombin und das Verhältnis von Fibrinogen zu Thrombin.

Durch entsprechende Steuerung kann eine hohe Reproduzierbarkeit der Fibrinsegmente als bioartifizielle Konstrukte (Gefäßprothese) mit einer homogenen Verteilung des Fibrins bei hohen biomechanischen Belastbarkeiten hergestellt werden.

In einem weiteren Aspekt werden entsprechend erhältliche bioartifizielle Konstrukte offenbart. Diese zeigen die bereits oben genannten Eigenschaften zur hohen biomechanischen Belastbarkeit bei homogener Verteilung und hoher Reproduzierbarkeit auf. Z. B. zeigten sie eine besonders gute Stabilität mit hohen Berstungsdrücken, sodass die erfindungsgemäßen bioartifiziellen Konstrukte auch für Arterien mit hoher Druckbelastung, insbesondere auch als Aorta-Prothese eingesetzt werden können.

In einer Ausführungsform ist das erfindungsgemäße bioartifizielle Konstrukt entsprechend eines, das eine Länge von mindestens 10 cm, wie mindestens 30 cm und eine Wanddicke von mindestens 1 mm aufweist. In den Ausführungsformen ist dabei das Konstrukt eine Gefäßprothese, insbesondere eine Prothese für eine Arterie. Bei einer Arterie beträgt die Wanddicke einer Gefäßprothese üblicherweise 10 bis 20 % des Durchmessers des Gefäßes.

In einem weiteren Aspekt richtet sich die vorliegende Erfindung auf eine Vorrichtung zur Herstellung von bioartifiziellen Konstrukten. Diese Vorrichtung umfasst eine erfindungsgemäße Einrichtung, i) einen Applikator zum Einbringen von Fluiden, üblicherweise die entsprechenden Proteinzusammensetzungen und Vernetzer-Zusammensetzungen in den Innenraum der inneren Form der erfindungsgemäßen Einrichtung; ii) eine Vorrichtung zum Druckbeaufschlagen der eingebrachten Fluide in die innere Form durch Rotation und iii) Zuleitungen zum Einbringen der Fluide über einen Applikator. Darüber hinaus kann die erfindungsgemäße Vorrichtung entsprechend eine Steuereinheit zum Steuern der Vorrichtung insbesondere der Parameter, wie sie oben bei der Herstellung genannt wurden, umfassen. Diese Schritte zur Herstellung umfassen dabei die Dauer des Einzelschrittes, der Rotationsgeschwindigkeit, der Form, der Position des Applikators, der jeweiligen Applikationswarte bei der Analyse und darüber hinaus das Verhältnis der beiden Komponenten, wie dem Fibrinogen zu Thrombin.

Darüber hinaus kann die erfindungsgemäße Vorrichtung entsprechende Vorratsbehälter für die einzubringenden Fluide aufweisen.

Schließlich wird in einem weiteren Aspekt eine Fibrinogen-Lösung, insbesondere zur Verwendung in dem erfindungsgemäßen Verfahren für bioartifizielle Konstrukte gestellt. Diese erfindungsgemäße Fibrinogen-Lösung ist dabei eine, die mindestens zwei unterschiedliche Fibrinogen-Lösungen umfasst, nämlich i) eine erste Fibrinogen-Lösung und ii) eine Fibrinogen-Lösung. Die erste Fibrinogen-Lösung ist eine, die im Wesentlichen frei von anderen Bestandteilen ist, z. B. eine, die sowohl Plasminogen deplettiert ist als auch deplettiert von anderen Bestandteilen, insbesondere Serum-Proteine, wie Albumin. Z. B. ist diese Lösung eine aufgereinigte Fibrinogen-Lösung, wie sie z. B. von Coachrom Diagnostic GmbH, Maria Enzersdorf, Österreich, angeboten wird.

Die zweite Fibrinogen-Lösung ist eine, die weiterhin nicht Fibrinogen-Proteine oder andere Moleküle aufweist. Diese zweite Fibrinogen-Zusammensetzung kann z. B. eine sein, wie sie üblicherweise durch Fibrinogen-Präzipitation erhalten wird, z. B. mittels Kryopräzipitation aus Blut bzw. Plasma separiert werden kann. Dieses sind z. B. Fibrinogen-Präparationen, die in einen automatisierten Prozess, z. B. VIVOSTAT^{™} separiert werden. Hierbei wird durch Zugabe bestimmter Detergenzien und Abkühlen auf niedrige Temperatur eine Trennung mittels Zentrifugal-Separation erreicht. Bei dieser Separation können aber auch andere Proteine aus dem Blut bzw. Plasma mit separiert werden. Es zeigte sich nun, dass eine Mischung dieser beiden unterschiedlichen Fibrinogen-Präparationen (teilweise bereits anpolymerisiert) die Herstellung von Prothesen erlaubte, die besonders gute mechanische Eigenschaften aufzeigen. Während Fibrinprothesen, hergestellt allein aus dem aufgereinigten Fibrinogen ohne weitere Bestandteile sehr hohe Berstungsdrücke aufzeigen, sind sie weniger elastisch insbesondere recht spröde, so dass sie als Prothesen nicht geeignet sind. Durch entsprechende Zumischung der Fibrinogen-Lösungen mit weiteren nicht-Fibrinogen Proteinen oder anderen Molekülen, können die Berstungsdrücke gesenkt werden, die Flexibilität und Elastizität nehmen zu, so dass arterielle Prothesen bereitgestellt werden können, die einen ausreichenden Berstungsdruck bei ausreichender Flexibilität aufzeigen. Die notwendige Flexibilität und Elastizität mit hohen Berstungsdrücken erlauben, geeignete Aorta-Prothesen bereitzustellen.

Die zweite Fibrinogen-Lösung kann, wie gesagt, weitere Moleküle, z. B. Proteine, enthalten, die bei der Separation durch Zentrifugation mit separiert werden. Alternativ oder zusätzlich können andere biologische und oder bioartifizielle Bestandteile vorliegen, die insbesondere nicht immunogen und im Allgemeinen inert sind. Der Ausdruck inert meint, dass die Moleküle keine immunogene Reaktion oder sonstige Reaktion im Körper eines Individuums auslösen. Z. B. können geeignete Substanzen vorliegen, um die entsprechende Flexibilität der Prothese zu beeinflussen, ohne immunologische Reaktionen im Empfängerorganismus auszulösen. D. h., durch die Zusammensetzung der Fibrinogen-Präparationen können die biomechanischen Eigenschaften der erhaltenen Prothesen gezielt beeinflusst werden. Während bei der Verwendung der aufgereinigten Fibrinogene die Fibrin-Fasern sehr dicht gelagert werden und es zu einer starken Quervernetzung kommt, können die Fibrinogen-Proteine oder andere Moleküle entsprechend diese Quervernetzung so beeinflussen, dass die Stabilität der verdichteten Matrix so groß wird wie in einer nativen Arterie. Durch die starke Quervernetzung ist die Sprödigkeit erhöht und die Elastizität verloren. Durch entsprechende Beimengung anderer Proteine oder anderer Moleküle, wie inerten Molekülen, können immunologische Reaktionen in den Empfängerorganismus verringert werden. Es ist daher vorteilhaft, entsprechende allogene Fibrinogen-Präparate einzusetzen.

In einer Ausführungsform ist die erfindungsgemäße Fibrinogen-Lösung aber eine Lösung, wobei die Verhältnisse der ersten Fibrinogen-Lösung und der zweiten Fibrinogen-Lösung in einem Bereich von 80:20 bis 20:80 liegen können. Je nach gewünschten, biomechanischen Eigenschaften werden die entsprechenden Verhältnisse eingestellt.

Darüber hinaus stellten die Erfinder fest, dass eine verbesserte Lagerung von Gefäßprothesen, wie erfindungsgemäßen bioartifiziellen Konstrukten, erreicht werden kann, wenn die einem Herstellungsprozess sich anschließende Dehydrierung, z. B. eine sich dem erfindungsgemäßen Verfahren zur Herstellung der bioartifiziellen Konstrukte anschließende Dehydrierung derart erfolgt, dass die entsprechende die Prothese ausbildende Matrix Flüssigkeit in einem Anteil von zwischen 20% bis 30% Feuchtigkeit aufweist, wie maximal 25 % aufweist.

Unverdichtetes Fibrin weist hohe viskoelastische Eigenschaften auf. Diese resultieren aus einem hohen Anteil von Flüssigkeit von ca. 80% in einem ungeordneten Geflecht von dreidimensional angeordneten Fibrinfasern, zwischen denen auch andere Proteine enthalten sind. Das Grundprinzip des vorliegenden Verfahrens besteht in der Reduktion der Flüssigkeit in der Fibrinmatrix. Durch die Herstellung in der Rotationsform wird der Flüssigkeitsanteil im Fibrin auf etwa 50 % reduziert. Eine weitere Reduktion durch verlängerte Zentrifugation erfolgt nicht im signifikanten Umfang. Eine Dehydrierung wurde bisher in trockener Atmosphäre (20 bis 30% Luftfeuchtigkeit) durchgeführt. Erfindungsgemäß konnte nun aufgezeigt werden, dass eine Dehydrierung in einer Klimakammer mit 80 % Luftfeuchtigkeit die Lagerbeständigkeit verbessert und die Stabilität der dehydrierten Prothesen verbessert ist. Während die Dehydrierung in trockener Atmosphäre, wie 20 bis 30 % Luftfeuchtigkeit, zu ungewünschten Ereignissen wie ein Aufreißen der Fibrin-Prothese oder eine andere Schädigung z. B. durch inhomogene Verteilung des Fibrins mit der Ausbildung lokaler Schwachstellen, geschieht, erfolgt die erfindungsgemäße Dehydrierung in einer Klimakammer mit 80 % Luftfeuchtigkeit schonender, so dass die Struktur der Fibrinmatrix verbessert ist. Selbst bei einer nicht vollständig homogenen Verteilung des Fibrins während der Herstellung in der Rotationsform geschah kein Aufreißen an möglichen Schwachstellen. Im Gegenteil scheint es nun so zu sein, dass bei einer nicht vollständig homogenen Verteilung des Fibrins während der Herstellung in der Rotationsform daraus resultierende Schwachstellen nicht durch eine zu schnelle Volumenreduktion des Fibrins zusätzlich belastet werden und dann reißen. Bei der Dehydrierung kommt es auch stets zu einer Volumenreduktion der Fibrinmatrix, die bei einer unter statischen Bedingungen generierten Fibrinmatrix zu der in der Literatur häufig beschriebenen Schrumpfung führt. Auch bei der Dehydrierung der in der Rotationsform hergestellten Fibrinsegmente kommt es zu einer Schrumpfung. Dieser Effekt wird vorliegend genutzt, indem dadurch Fibrinprothesen für kleine Gefäße mit einem inneren Durchmesser <5 mm hergestellt werden können. Dabei führt die Volumenreduktion durch den Flüssigkeitsentzug nur zu einer Schrumpfung im Durchmesser der Fibrinsegmente nicht aber in der Länge. Erfolgt die Dehydrierung nun langsam in einer feuchten Atmosphäre mit 80% Luftfeuchtigkeit wird eine Belastung von Schwachstellen in der Matrix durch eine zu schnelle Schrumpfung nicht nur vermieden. Vielmehr scheint sogar ein Ausgleich solcher Schwachstellen durch eine passive Umverteilung des Fibrins möglich zu sein.

Darüber hinaus ist es vorteilhaft bei der Rehydrierung, einen zusätzlichen Schritt des in Kontaktbringens mindestens der Enden, die den Anastomosenbereich darstellen, der Prothese mit Lösungen der Proteine, die zur Herstellung der bioartifiziellen Konstrukte eingesetzt wurden, wie mit Fibrinogen-Lösung durchzuführen.

Strukturanalysen der erfindungsgemäß hergestellten Gefäßprothesen zeigten für z.B. Fibrinprothesen, dass die Zunahme der Stabilität u.a. auf eine zunehmend parallele Anordnung der Fibrinfasern zurückzuführen ist. Diese richten sich während der Zentrifugation in der rotierenden Form und der anschließenden Dehydrierung zunehmend parallel zur Längsachse der Fibrinprothese aus. So führt eine mechanische Belastung zu einer Anspannung der Fasern in ihrer Längsrichtung, wo sie eine erheblich höhere Belastbarkeit haben als die Quervernetzung zwischen den Fasern. Durch die Lastverteilung des intraluminalen Drucks auf die Wand in einem zylindrischen Gefäß lässt sich daher gut erklären, warum dann auch der maximale Berstungsdruck der Fibrinsegmente signifikant ansteigt. Denn eine Erhöhung des intraluminalen Drucks in einem zylindrischen Gefäß führt vor allem zu einer Zunahme der Wandspannung in Längsrichtung des Gefäßes und weniger in Querrichtung.

Die parallele Anordnung der Fibrinfasern erklärt dabei nicht nur den signifikanten Anstieg der biomechanischen Belastbarkeit, sondern erklärt auch, warum die Nahtstabilität erheblich geringer ansteigt als maximale Wandspannung und Berstungsdruck. Ausfällig war aber, dass der Berstungsdruck Beispiel etwa um den Faktor 40 gesteigert werden konnte, die Nahtstabilität aber nur um das 4- bis 5-fache. Während bei einer Zunahme der Wandspannung in Längsrichtung des Gefäßes, wie sie eben auch bei einem Anstieg des intraluminalen Drucks entsteht, die Fibrinfasern in ihrer Längsrichtung angespannt werden, bilden sie dagegen kein ausreichendes Widerlager für eine Naht. Die mechanische Belastung auf eine Naht verläuft typischerweise auch in Längsrichtung eines Gefäßes, so wird ein Bypassmaterial typischerweise mit beiden Enden an die beiden Zielgefäße anastomosiert. So entsteht also bereits eine Zugbelastung auf die Naht in Längsrichtung des Gefäßes. Und auch der intraluminale Druck führt zu einer Belastung der Anastomose in Längsrichtung des Gefäßes. Die Zugbelastung einer Naht verläuft also jeweils in Längsrichtung der Fibrinprothese. Hier bilden aber nur die Querverbindungen zwischen den Fibrinfasern ein Widerlager für die Naht nicht aber die Fasern selbst.

Erfindungsgemäß erfolgt die Rehydrierung einer dehydrierten (getrockneten) Gefäßprothese, wie von Fibrinsegmenten nicht mehr wie bisher in einer isotonen Salzlösung, sondern in einer niedrig konzentrierten Lösung der Proteine, die zur Herstellung der bioartifiziellen Konstrukte eingesetzt wurden, wie z.B. einer Kollagenlösung, einer Fibrinlösung oder Mischungen hiervon, beispielhaft einer niedrig konzentrierten Fibrinogenlösung (25 mg/ ml). Dabei entsteht außen eine zusätzliche Schicht aus Fibrin und/oder Kollagen. Obwohl diese nur dünn ist und aus nicht verdichtetem Fibrin besteht, führt sie zu einer signifikanten Verbesserung der biomechanischen Eigenschaften der Fibrinprothese. So wird die Nahtstabilität signifikant gesteigert, so dass die Fibrinprothesen nun sogar an den Aortenbogen anastomosiert werden können, ohne dass es im Verlauf zu einer Ruptur kommt. Dabei stellt der Aortenbogen den Bereich mit der höchsten mechanischen Belastung im arteriellen Gefäßsystem dar. Dass eine Prothese aus Fibrin in diesem Bereich anastomosiert werden kann, ohne dass es auch im Verlauf zu einer Ruptur kommt, ist bisher nicht beschrieben worden. Außerdem führt die Rehydrierung in einer Fibrinogenlösung zu einer signifikanten Verbesserung der Elastizität und Flexibilität der Fibrinprothese.
Die Erfindung wird anhand von den beigefügten Figuren weiter erläutert.

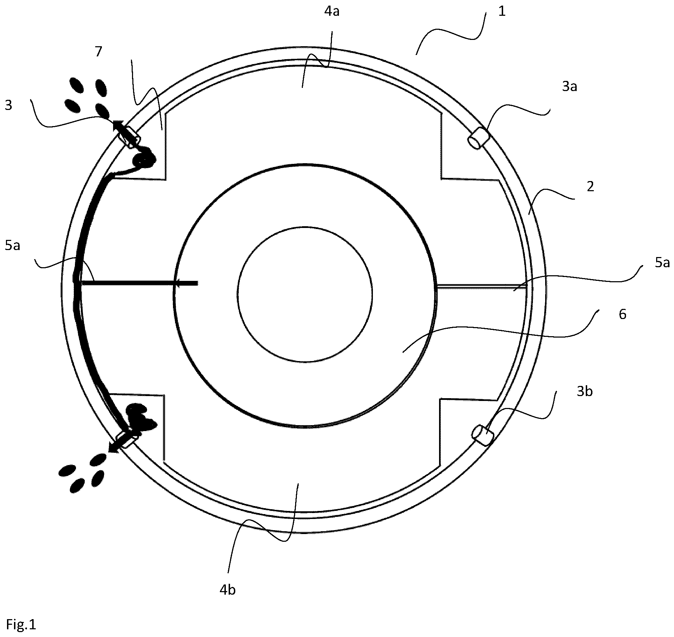

In der Figur 1 ist der Aufbau einer erfindungsgemäßen Einrichtung 1 im Querschnitt gezeigt. Hierbei ist eine äußere Hülle 2 mit Bohrungen 3, 3a, 3b zu sehen. In diese äußere Hülle ist eine innere Form 4 eingeschoben, diese innere Form 4 weist im vorliegenden Fall zwei Halbschalen 4a und 4b auf. Zwischen den Halbschalen 4a und 4b finden sich Kapillarspalten 5a und 5b über die Fluide aus dem Inneren der inneren Form 4 in den Zwischenraum zwischen innerer Form 4 und äußerer Form 5 fließen. Die Gefäßprothese 6, vorliegend eine Fibrin-Prothese, verbleibt im Inneren der inneren Form 4. Die durch die Kapillarspalten 5a und 5b austretende Flüssigkeit wird über die Bohrungen 3 nach außen geführt. Die Kavitäten 7 erlauben die Verbindung der beiden Halbschalen mittels Befestigungselementen (nicht dargestellt). Durch entsprechende Einstellung z. B. mit einer definierten Kraft miteinander Verschrauben der beiden Halbschalen, erfolgt die Ausbildung und Dicke der kapillären Spalten 5a und 5b.

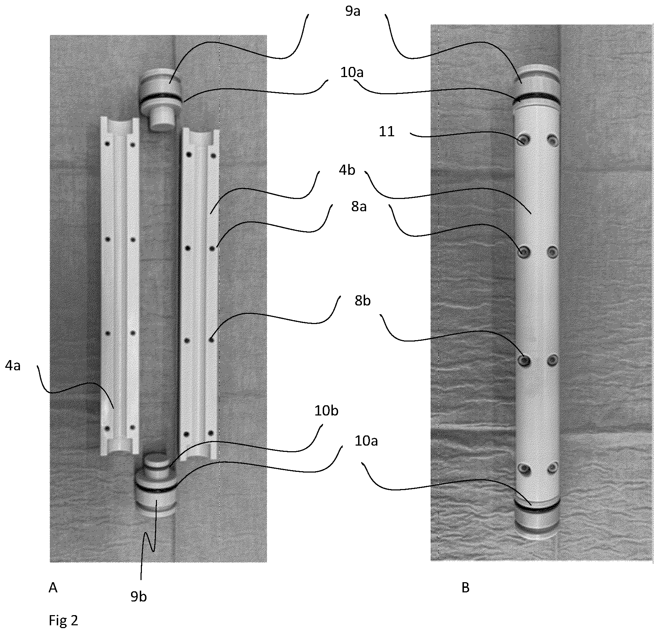

In der Figur 2a sind die beiden Halbschalen 4a und 4b dargestellt. Vorliegend wurden diese beiden Halbschalen 4a und 4b aus PEEK hergestellt. Zu sehen sind ebenfalls die Bohrungen 8a, 8b, ... in den Halbschalen zur Aufnahme der Befestigungselemente, wie den Schrauben. Ebenfalls zu sehen sind die stirnseitig angeordneten lösbaren Abschlusselemente 9, 9a, 9b mit entsprechenden Dichtungselementen 10, 10a, 10b zum Verschließen der inneren Form und zum Abdichten der äußeren Hülle.

In der Figur 2b ist eine zusammengefügte innere Form mit den stirnseitig vorgegebenen Abschlusselementen dargestellt. Die Halbschalen 4a und 4b sind mit Schrauben 11, die in den Bohrungen 8a, 8b ... vorliegen, miteinander verbunden derart, dass kapillare Spalten (nicht dargestellt) zwischen diesen beiden Halbschalen vorliegen. Die Abschlusselemente 9a, 9b, zeigen die Dichtungselemente 10a und 10b zum Abdichten des Zwischenraumes zwischen der inneren Form und der äußeren Form bei Einschieben in die äußere Hülle auf.

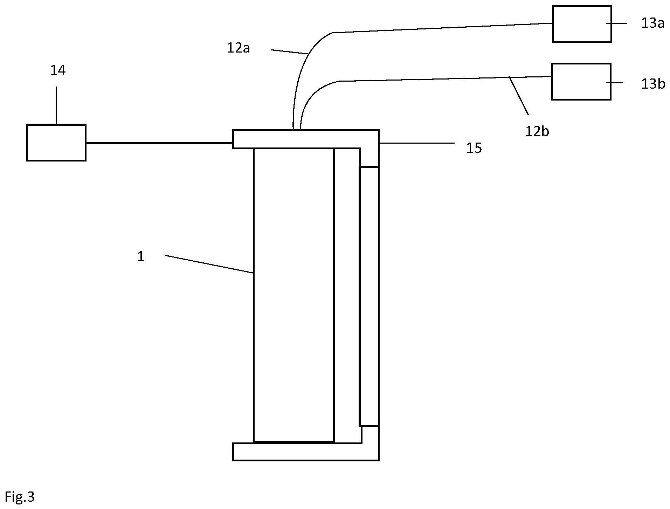

In der Figur 3 ist eine erfindungsgemäße Vorrichtung schematisch dargestellt. Diese umfasst die erfindungsgemäße Einrichtung 1 mit einer Vorrichtung zum Druckbeaufschlagen der eingebrachten Fluide in die innere Form durch Rotation 15 und Zuleitungen 12; 12a, 12b, zum Einbringen von Fluiden über einen Applikator in die Einrichtung 1. Der Applikator ist nicht dargestellt, er erstreckt sich über das Abschlusselement mit Durchgangsöffnung in das Innere der inneren Form der erfindungsgemäßen Einrichtung 1. Die Zuleitungen 12a, 12b zum Einbringen der Fluide über einen Applikator sind verbunden mit den Vorratsbehältern 13a und 13b, die entsprechende Fibrinogen-Lösung bzw. Fibrinogen-Zusammensetzung der einzelnen Fibrinogen-Lösungen, und eine Thrombin-Lösung aufweisen. Die Steuerung der Druckbeaufschlagung des Einbringens der Fluide über den Applikator erfolgt über die Steuereinheit 14.

Die Erfindung wird weiterhin anhand von Beispielen im Folgenden erläutert:

### Beispiel 1 Herstellung einer Gefäßprothese mit einer erfindungsgemäßen Einrichtung

Fibrinogen wird aus FFP (Fresh frozen Plasma) mittels eines Gefrier-/Auftauzyklus separiert. Plasma von 100 Spendern wird gepoolt, um interindividuelle Unterschiede in der Plasmazusammensetzung auszugleichen.

Das Plasma wird aliquotiert in 50ml Gefäßen und für 24 Stunden bei -20°C eingefroren. Dann wird das gefrorene Plasma bei +3°C wieder aufgetaut und anschließend für 45 min bei 3.000 g und 3°C zentrifugiert. Das dabei entstehende Fibrinogen-Pellet wird zweimal mit 3°C kaltem Aqua gewaschen und nach jedem Waschgang für 10 min bei 3.000 g und 3°C zentrifugiert.

Auf das entstandene Fibrinogen-Pellet wird anschließend in 3°C kaltem destillierte Wasser resuspendiert und für 1 Stunde bei 37°C inkubiert.

Zu Herstellung der Fibrinprothese werden zwei getrennte Lösungen hergestellt:
**Lösung 1 (für 70/30 Mischverhältnis) Beispiel**

| | |
|---|---|
| 500 mg | Kommerziell erwerbliches Fibrinogen (CoaChrom Diagnostica GmbH) |
| 200 mg | Cryopräzipiertes Fibrinogen |
| 100 U/ml | Aprotinin |

**Lösung 1 (für 50/50 Mischverhältnis) Beispiel**

| | |
|---|---|
| 350 mg | Kommerziell erwerbliches Fibrinogen (CoaChrom Diagnostica GmbH) |
| 350 mg | Cryopräzipiertes Fibrinogen |
| 100 U/ml | Aprotinin |

**Lösung 2 (Thrombinlösung)**

| | |
|---|---|
| 3500 µl | 40 mM Kalziumchlorid-Lösung |
| 1600 µl | Faktor XIII |
| 300 µl | Thrombin |

Lösung 1 enthält kommerziell erwerbliches Fibrinogen (CoaChrom Diagnostica GmbH, Maria Enzersdorf, Österreich) und kryopräzipitiertes Fibrinogen aus FFP. Für ein 20 cm langes Segment werden insgesamt 700 mg Fibrin benötigt. Das Mischungsverhältnis beträgt 70 % aufgereinigtes Fibrinogen und 30 % kryopräzipitiertes Fibrinogen. Das zweite Mischungsverhältnis beträgt 50 % aufgereinigtes Fibrinogen und 50 % kryopräzipitiertes Fibrinogen mit zusätzlich Aprotinin (100 U/ml).

Lösung 2 ist eine 40 mmolare Kalziumchlorid-Lösung, die 100 bis 200 (150) Thrombin und 40 bis 100 (60) Einheiten Faktor XIII (Fibrogammin) enthält.

Die PEEK Halbschalen (Länge: 200 mm; innerer Durchmesser: 7 mm) werden zunächst mit sterilem Fett eingefettet. An beiden Enden der Halbschalenform werden Abschlusselemente eingelegt und die Form zusammengeschraubt. Das obere Abschlusselement hat ein 4mm großes Loch über das die beiden Lösungen mithilfe einer Kanüle (20G; 70mm) in die Form gespritzt werden können. Die zusammen gefügten Halbschalen werden in die äußere Metallröhre der Form gesteckt.

Die beiden Lösungen werden jeweils in eine 20 ml Spritze steril aufgezogen. Anschließend werden die Spritzen mit je einer Silikonleitung mit einem Y-Konnektor verbunden, welches am anderen Ende mit einer 20G Kanüle (70 mm) verbunden ist. Die beiden Lösungen werden simultan mit definierte Flussgeschwindigkeiten in die sich mit hoher Geschwindigkeit drehende Form appliziert.

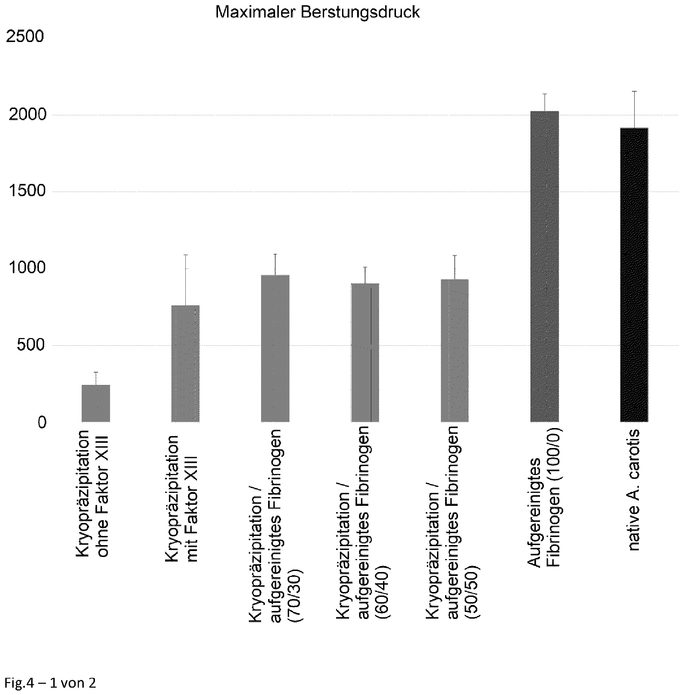

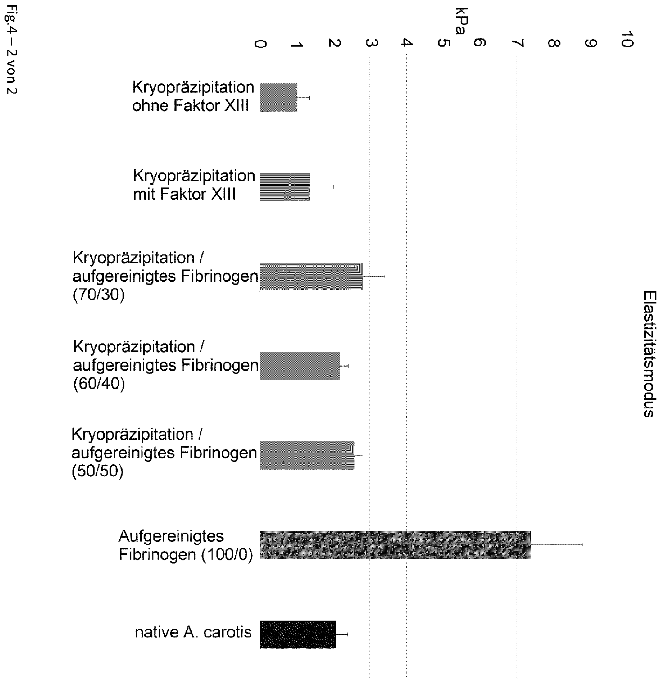

Figur 4: Figur 4 zeigt die Messungen zum Berstungsdruck und Elastizitätsmodulus wie sie z.B. in Regenberg 1M.-C. et al., J Mech Behav Biomed Mater. 2023 Dec: 148:106199 beschrieben sind. Es konnten mit der alleinigen Verwendung aufgereinigten Fibrinogens Fibrinprothesen mit einem maximalen Berstungsdruck von 2.000 mmHg hergestellt werden - also entsprechend dem einer nativen Arterie. Allerdings waren diese so wenig flexibel, dass sie nicht als Bypassmaterial verwendet werden können. Durch die Mischung des aufgereinigten Fibrinogens mit kryopräzipitiertem Fibrinogen, das neben Fibrinogen etwa 250 weitere Proteine enthält, sinkt zwar die maximale Stabilität der daraus hergestellten Fibrinprothesen. Dafür sind sie deutlich flexibler und elastischer. Die geringe Standardabweichung zeigt auch an, mit welcher Reproduzierbarkeit die Fibrinprothesen (jeweils n=10) hergestellt werden konnten. Es wurden verschiedene, in der Figur dargestellte Mischungsverhältnisse untersucht.

### Beispiel 2 Dehydrierung

Die generierte Fibrinprothese wird für eine Stunde in 70% Ethanol gebadet. Danach wird die Fibrinprothese dreimal für 10 min mit PBS (1x) gewaschen. Anschließend wird ein Dilatator mit gewünschter Größe (2-4 mm) in die Fibrinprothese eingeführt. Die Fibrinprothese wird in eine Klimakammer gelegt, in der eine definierte Luftfeuchtigkeit von 80% bei Raumtemperatur herrscht. In dieser wird sie für mindestens 7 Tage dehydriert.

### Beispiel 3 Rehydrierung

Nach Ablauf der Dehydrierung wird die Fibrinprothese für 11 Tage in PBS mit 1x Penicillin/Streptomycinlösung und Aprotinin (100U/ml) in einem Messzylinder stehend rehydriert (Stand der Technik).

Für eine alternative Rehydrierung zur Aufbringung einer äußeren unverdichteten Fibrinschicht wird die dehydrierte Fibrinprothese, in der sich noch der Dilatator befindet in eine tubuläre Form gesteckt, deren Durchmesser 2 bis 3 mm größer ist als der äußere Durchmesser der Fibrinprothese. Zuvor wurde die tubuläre Form, die unten verschlossen ist, etwa zu einem Drittel mit einer Fibrinogenpräparation aus kryopräzipitiertem Fibrinogen gefüllt ist. Über eine Vertiefung im Verschluss des unteren Endes der Form und einen Cuff, der in das obere Ende eingebracht wird, wird der Dilatator in der Fibrinprothese so fixiert, dass die Fibrinprothese mittig in der senkrecht stehenden Form steht. Die Form mit der Fibrinprothese wird dann über 12 Stunden in einem Inkubator mit 37°C inkubiert.

Jeweils am Ende der Rehydrierung kann der Dilatator aus der Fibrinprothese herausgezogen werden.

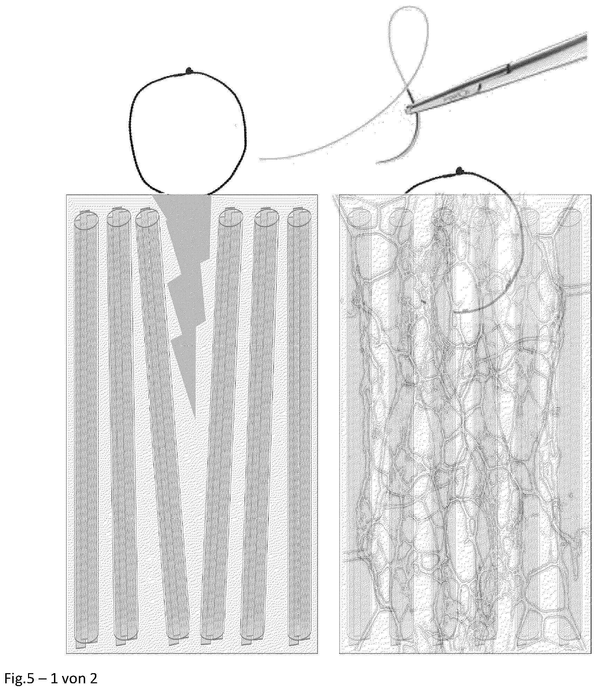

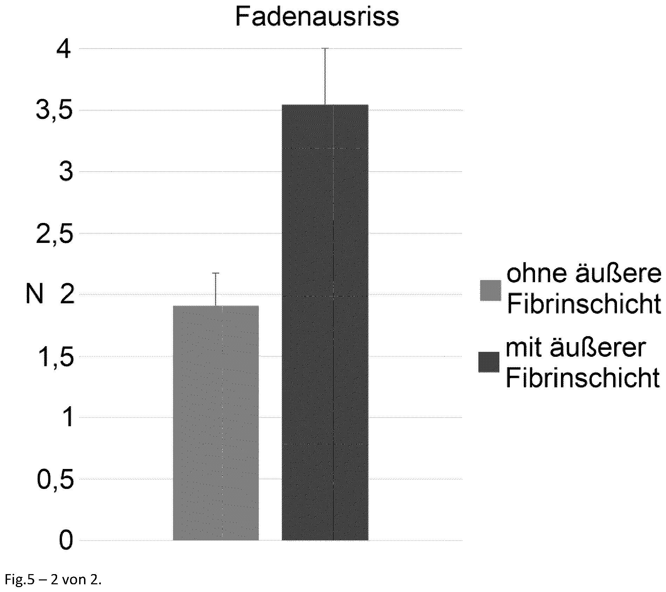

Figur 5 zeigt auf der linken Seite die Annahme, dass durch die parallele Anordnung der Fasern die Naht dann vor allem die Querverbindungen zwischen den Fasern als Nahtlager fehlen und dann ausreißt. Durch die Modifikation des Herstellungsprozesses, bei dem das dehydrierte Fibrin in einer Fibrinogenlösung rehydriert wird, was zur Aufbringung einer äußeren Schicht aus unverdichtetem Fibrin führt, die sich aber gut mit der Wand der Fibrinprothese verbindet ("Fibhyd"), konnte die Nahtstabilität signifikant gesteigert werden (rechte Skizze auf der linken Seite und Diagramm rechts). Die Fibrinprothesen konnten problemlos sogar an den Aortenbogen anastomosiert werden, wo eine hohe mechanische Belastung auf die Anastomose und die Fibrinprothese einwirkt.

### Bezugszeichenliste

- 1: Einrichtung
- 2: Äußere Hülle
- 3, 3a, 3b,..: Bohrungen
- 4, 4q, 4b, ..: innere Form
- 5, 5a, 5b, ..: Kapillarspalten
- 6: Gefäßprothese
- 7: Kavitäten
- 8, 8a, 8b,...: Bohrungen
- 9: Abschlusselement
- 10, 10a, 10b,..: Dichtungselemente
- 11: Schraube
- 12, 12a, 12b,..: Zuleitungen
- 13, 13a, 13b,..: Vorratsbehälter
- 14: Steuereinheit
- 15: Rotationsvorrichtung

## Patentansprüche

1. Einrichtung, geeignet zur Herstellung von bioartifiziellen Konstrukten auf Proteinbasis, wie auf Kollagenbasis oder Fibrinbasis durch ein Rotationsverfahren, mit i) einer äußeren Hülle und ii) einer inneren Form, wobei die innere Form in die äußere Hülle eingeführt werden kann und die innere Form stirnseitig angesetzte, lösbare Abschlusselemente aufweist, die äußere Hülle Bohrungen zum Abführen von Fluiden umfasst und die innere Form mindestens zweiteilig ist und mit Befestigungselementen zum lösbaren Verbinden der mindestens zweiteiligen inneren Form vorliegt, **dadurch gekennzeichnet, dass** die mindestens zweiteilige innere Form durch die Befestigungselemente derart verbunden vorliegt, dass Fluide durch die in den mindestens zwei schalenförmigen Teilen der inneren Form ausgebildeten Spalten aus dem Inneren der inneren Form in den Zwischenraum zwischen innerer Form und äußerer Hülle fließen können und diese Fluide dann durch die Bohrungen der äußeren Hülle nach außen abgeführt werden können.

2. Einrichtung nach Anspruch 1, wobei sich die innere Form und die äußere Hülle in eine Längsrichtung erstreckt und/oder die innere Form und äußere Hülle röhrenförmig, insbesondere zylindrisch sind, und koaxial angeordnet sind, bevorzugt wobei die innere Form als mindestens zweiteilige schalenförmige Teile ausgebildet ist, wobei der Querschnitt im Wesentlichen kreisförmig ist und der Querschnitt der inneren Form kleiner ist als der Querschnitt der äußeren Hülle zum Einschieben der inneren Form in die äußere Hülle.

3. Einrichtung nach Anspruch 1 oder 2, wobei mindestens ein Abschlusselement eine Durchgangsöffnung zum Einbringen von Fluiden in das Innere der inneren Form aufweist.

4. Einrichtung nach einem der vorherigen Ansprüche, wobei die innere Form keine Bohrung aufweist und/oder die äußere Hülle Bohrungen von mindestens 1 mm, bevorzugt mindestens 2 mm, verteilt aufweist.

5. Einrichtung nach einem der vorherigen Ansprüche, wobei die Spalten zwischen den mindestens zwei schalenförmigen Teilen der inneren Form Kapillarspalten sind.

6. Einrichtung nach einem der vorherigen Ansprüche, wobei die innere Form und äußere Form sowie Abschlusselemente sterilisierbar sind, geeignet zur Herstellung von bioartifiziellen Medizinprodukten.

7. Verfahren zur Herstellung von bioartifiziellen Konstrukten auf Proteinbasis mit den Schritten:
a) Bereitstellen von proteinhaltigen Zusammensetzungen und Vernetzer-haltigen Zusammensetzungen;
b) Einbringen der proteinhaltigen Zusammensetzungen und der Vernetzer-haltigen Zusammensetzungen in die Einrichtung nach einem der Ansprüche 1 bis 6 unter Druckbeaufschlagung dieser Einrichtung, wobei die Druckbeaufschlagung durch Rotation der Einrichtung erfolgt und wobei während des Rotationsverfahrens Fluide über die Bohrungen der äußeren Hülle abgeführt werden;
c) Entfernen des bioartifiziellen Konstruktes auf Proteinbasis aus der inneren Form der Einrichtung nach einem der Ansprüche 1 bis 6.

8. Verfahren nach Anspruch 7 zur Herstellung von bioartifiziellen Konstrukten auf Fibrinbasis mit den Schritten:
a) Bereitstellung von Fibrinogen-haltigen Zusammensetzungen und Thrombinhaltigen Zusammensetzungen;
b) Einbringen der Fibrinogen-haltigen Zusammensetzungen und der Thrombinhaltigen Zusammensetzungen in die Einrichtung nach einem der Ansprüche 1 bis 6 unter Druckbeaufschlagung dieser Einrichtung, wobei die Druckbeaufschlagung durch Rotation der Einrichtung erfolgt und wobei während des Rotationsverfahrens Fluide über die Bohrungen der äußeren Hülle abgeführt werden;
c) Entfernen des bioartifiziellen Konstruktes auf Fibrinbasis aus der inneren Form der Einrichtung nach einem der Ansprüche 1 bis 6.

9. Verfahren zur Herstellung von bioartifiziellen Konstrukten nach Anspruch 7 oder 8, wobei die Druckbeaufschlagung durch Rotation, insbesondere einer Rotation, dass an der Oberfläche mindestens 100 g, wie mindestens 200 x g, wie mindestens 500 x g.

10. Verfahren zur Herstellung eines bioartifiziellen Konstruktes nach einem der Ansprüche 7 bis 9, wobei die Fibrinogen-Lösung eine ist aus einer Mischung umfassend i) Fibrinogen, das frei von weiteren Bestandteilen ist, und, ii) Fibrinogen enthaltend weitere Bestandteile insbesondere Serumalbumin.

11. Verfahren zur Herstellung eines bioartifiziellen Konstruktes nach einem der Ansprüche 7 bis 10, wobei dieses Konstrukt eine Wanddicke von mindestens 0,4 mm wie mindestens 1 mm aufweist.

12. Bioartifizielles Konstrukt, erhältlich mit einem Verfahren nach einem der Ansprüche 7 bis 11, insbesondere wobei dieses eine Länge von mindestens 10 cm, wie mindestens 30 cm und eine Wanddicke von mindestens 1 mm aufweist, bevorzugt wobei dieses ein Gefäß, insbesondere eine Prothese für eine Ader, wie eine Arterie ist.

13. Vorrichtung zur Herstellung von bioartifiziellen Konstrukten umfassend i) eine Einrichtung nach einem der Ansprüche 1 bis 6, ii) einen Applikator zum Einbringen von Fluiden in den Innenraum der inneren Form der Einrichtung nach einem der Ansprüche 1 bis 6; iii) eine Vorrichtung zum Druckbeaufschlagen der eingebrachten Fluide in die innere Form durch Rotation; iv) Zuleitungen zum Einbringen der Fluide über einen Applikator, bevorzugt, weiterhin umfassend eine Steuereinheit zum Steuern der Druckbeaufschlagung und des Einbringens der Fluide in die Einrichtung und/oder weiter umfassend Vorratsbehälter für die einzubringenden Fluide.

14. Fibrinogen-Lösung insbesondere zur Verwendung in der Herstellung von bioartifiziellen Konstrukten, wobei die Fibrinogen-Lösung eine ist, umfassend i) eine erste Fibrinogen-Lösung und ii) eine zweite Fibrinogen-Lösung, wobei die erste Fibrinogen-Lösung eine ist, die im Wesentlichen frei von anderen Proteinen ist und wobei die zweite Fibrinogen-Lösung eine ist, die weiterhin Nicht-Fibrinogen-Proteine oder andere Moleküle enthält.

15. Fibrinogen-Lösung nach Anspruch 15, wobei die Verhältnisse der ersten Fibrinogen-Lösung und der zweiten Fibrinogen-Lösung in einem Bereich von 80 : 20 bis 20 : 80 liegen.
